# EUROPEAN PATENT SPECIFICATION

(11) **EP 1 888 033 B1**
(45) Date of publication and mention of the grant of the patent: **19.02.2014**
(21) Application number: 06744143.6
(22) Date of filing: 08.06.2006
(51) Int. Cl.: A61K 9/127, A61P 11/02, A61P 11/06, A61P 29/00, A61K 31/55, A61K 31/58, A61K 31/381, A61K 31/56, A61K 31/415, A61K 31/405

(54) **METHOD AND COMPOSITION FOR TREATING INFLAMMATORY DISORDERS**
VERFAHREN UND ZUSAMMENSETZUNG ZUR BEHANDLUNG VON ENTZÜNDLICHEN ERKRANKUNGEN
METHODE ET COMPOSITION DE TRAITEMENT DE TROUBLES INFLAMMATOIRES

(30) Priority: 09.06.2005 US 688698 P; 07.07.2005 US 696777 P
(43) Date of publication of application: 20.02.2008
(73) Proprietor: Meda AB, 170 09 Solna (SE)
(72) Inventor: PERESWETOFF-MORATH, Lena, 751 05 Uppsala (SE); CARLSSON, Anders, 112 38 Stockholm (SE)
(74) Representative: Endler, Gabriele
(86) International application number: PCT/GB2006/002090
(87) International publication number: WO 2006/131737

(56) References cited:
- WO-A-90/14105
- WO-A-96/40090
- WO-A-98/00111
- WO-A-2005/010001
- US-A- 4 427 649
- US-A- 4 839 175
- US-A- 5 455 271
- US-A- 5 569 464
- US-A- 5 703 093
- US-A- 5 792 776
- US-A1- 2002 064 524
- US-A1- 2003 054 030
- IWANAGA K ET AL: "USEFULNESS OF LIPOSOMES AS AN INTRANASAL DOSAGE FORMULATION FOR TOPICAL DRUG APPLICATION" BIOLOGICAL & PHARMACEUTICAL BULLETIN (OF JAPAN), PHARMACEUTICAL SOCIETY OF JAPAN, TOKYO, JP, vol. 23, no. 3, March 2000 (2000-03), pages 323-326, XP008056826 ISSN: 0918-6158
- GURSOY A: "PREPARATION, CHARACTERIZATION AND ANTI-INFLAMMATORY EFFECT OF DEFIBROTIDE LIPOSOMES" PHARMAZIE, DIE, GOVI VERLAG, ESCHBORN, DE, vol. 48, no. 7, 1 July 1993 (1993-07-01), pages 549-550, XP000372658 ISSN: 0031-7144
- VAN BALEN GEORGETTE PLEMPER ET AL: "Lipophilicity behaviour of the zwitterionic antihistamine cetirizine in phosphatidylcholine liposomes/water systems" PHARMACEUTICAL RESEARCH, NEW YORK, NY, US, vol. 18, no. 5, May 2001 (2001-05), pages 694-701, XP002357154 ISSN: 0724-8741
- ELZAINY ABEER A W ET AL: "Hydroxyzine from topical phospholipid liposomal formulations: evaluation of peripheral antihistaminic activity and systemic absorption in a rabbit model." AAPS PHARMSCI [ELECTRONIC RESOURCE]. 5 NOV 2003, vol. 5, no. 4, 5 November 2003 (2003-11-05), page E28, XP002361750 ISSN: 1522-1059
- PAULUSSEN J J ET AL: "Influence of the antiallergic drug oxatomide and derivatives on membrane structures: relation with inhibition of calcium influx in rat basophilic leukemia cells." BIOCHEMICAL PHARMACOLOGY. 1 MAR 1999, vol. 57, no. 5, 1 March 1999 (1999-03-01), pages 503-510, XP002361751 ISSN: 0006-2952
- AHMED M ET AL: "Partitioning and efflux of phenothiazines from liposomes" BIOCHEMICAL PHARMACOLOGY 1980 UNITED KINGDOM, vol. 29, no. 17, 1980, pages 2361-2365, XP002361752
- MISHINA E V ET AL: "INHIBITION OF RAT SPLENOCYTE PROLIFERATION WITH METHYLPREDNISOLONE: IN VIVO EFFECT OF LIPOSOMAL FORMULATION" PHARMACEUTICAL RESEARCH, NEW YORK, NY, US, vol. 11, no. 6, June 1994 (1994-06), pages 848-854, XP009000853 ISSN: 0724-8741
- WELCH M J: "New-generation antihistamines." THE WESTERN JOURNAL OF MEDICINE. APR 1991, vol. 154, no. 4, April 1991 (1991-04), page 455, XP002361753 ISSN: 0093-0415
- WILSON ANDREW M ET AL: "Leukotriene receptor antagonists for allergic rhinitis: a systematic review and meta-analysis." THE AMERICAN JOURNAL OF MEDICINE. 1 MAR 2004, vol. 116, no. 5, 1 March 2004 (2004-03-01), pages 338-344, XP002369671 ISSN: 0002-9343
- VEMURI S ET AL: "Preparation and characterization of liposomes as therapeutic delivery systems: a review" PHARMACEUTICA ACTA HELVETIAE, XX, XX, vol. 70, no. 2, July 1995 (1995-07), pages 95-111, XP002357174 ISSN: 0031-6865

## Description

### Field of the Invention

This invention relates to compositions for use in methods of treating inflammatory disorders.

### Background and Prior Art

There are many diseases/disorders that are inflammatory in their nature. Inflammatory diseases that affect the population include asthma, inflammatory bowel disease, rheumatoid arthritis, osteoarthritis, rhinitis, conjunctivitis and dermatitis.

Inflammation is also a common cause of pain. Inflammatory pain may arise for numerous reasons, such as infection, surgery or other trauma. Moreover, several diseases including malignancies and cardiovascular diseases are known to have inflammatory components adding to the symptomatology of the patients.

Asthma is a disease of the airways that contains elements of both inflammation and bronchoconstriction. Treatment regimens for asthma are based on the severity of the condition. Mild cases are either untreated or are only treated with inhaled [beta]-agonists which affect the bronchoconstriction element, whereas patients with more severe asthma are typically treated regularly with inhaled corticosteroids which to a large extent are antiinflammatory in their nature. A new preventative therapy for asthma works by blocking the production of proinflammatory leukotrienes and cytokines through inhibiting the 5-lipoxygenase enzyme.

Allergic and non-allergic rhinitis are common disorders affecting about 30% of the population. Rhinitis has a considerable impact on quality of life. In fact, rhinitis is regarded to affect the quality of life more so than, e.g., asthma.

Hay fever and perennial allergic rhinitis are characterised by sneezing, rhinorrhea, nasal congestion, pruritus, conjunctivitis and pharyngitis. In perennial rhinitis, chronic nasal obstruction is often prominent and may extend to eustachian tube obstruction.

Oral or local antihistamines are first line treatments, and nasal steroids second line treatments for rhinitis. For most patients, topical corticosteroids and long acting antihiistamine agents provide significant relief of symptoms. Antihistamines may also affect non-immunologically (non-IgE) mediated hypersensitivity reactions such as non-allergic rhinitis, exercise induced asthma, cold urticaria, and nonspecific bronchial hyperreactivity.

The main clinical effects of antihistamines include reduced sneezing and rhinorrhea. However, nasal blockage appears to be less responsive. Local administration of antihistamines (such as azelastine and levocabastine) has advantages, including rapid onset of action and fewer side effects.

Inflammatory pain may be reduced by the inhibition of the cyclooxygenase (COX) enzyme. The COX enzyme exists in two forms, one that is constitutively expressed in many cells and tissues (COX-I), and one that is induced by proinflammatory stimuli, such as cytokines, during an inflammatory response (COX-2).

COXs metabolise arachidonic acid to the unstable intermediate prostaglandin H2 (PGH2). PGH2 is further metabolized to other prostaglandins including PGE2, PGF2(X5 PGD2, prostacyclin and thromboxane A2. These arachidonic acid metabolites are known to have pronounced physiological and pathophysiological activity including proinflammatory effects.

PGE2 in particular is known to be a strong proinflammatory mediator, and is also known to induce fever and pain. Consequently, numerous drugs have been developed with a view to inhibiting the formation of PGE2, including "NSAIDs" (non-steroidal antiinflammatory drugs) and "coxibs" (selective COX-2 inhibitors).

These drugs act predominantly by inhibition of COX-1 and/or COX-2, thereby reducing the formation of PGE2.

The leukotrienes (LTs) are formed from arachidonic acid by a set of enzymes distinct from those in the COX / PGES pathway. The key enzyme in leukotriene biosynthesis is 5 -lipoxygenase (5-LO), which in a two-step reaction catalyzes the formation of LTA₄ from arachidonic acid. Leukotriene A₄ can be further metabolized into Leukotriene B₄, a reaction catalyzed by LTA₄ hydrolase. Cellular leukotriene biosynthesis is dependent on 5 -lipoxygenase activating protein (FLAP), a membrane bound protein which binds arachidonic acid and facilitates the 5-lipoxygenase reaction. Leukotriene B4 is known to be a strong proinflammatory mediator, while the cysteinyl-containing leukotrienes C₄, D₄ and E₄ (CysLTs) are very potent bronchoconstrictors and proinflammatory mediators that have been implicated in the pathobiology of asthma and inflammation. Therefore, the marketed 5-LO inhibitors and antagonists of cystenyl-containing leukotriene receptors 1 and 2 represent two new classes of anti-inflammatory treatments, while the development of marketed FLAP inhibitors, leukotriene A₄ hydrolase inhibitors, leukotriene B₄ receptor antagonists may provide further new classes of anti-inflammatory treatments.

Phosphodiesterase type 4 (PDE 4) plays an important role in modulating the activity of cells that are involved in the inflammatory processes that occur in chronic obstructive pulmonary disorder (COPD) and asthma. PDE4 inhibitors represent a new class of drugs that have the potential to inhibit bronchoconstriction as well as inhibit inflammatory cell activity (including inhibiting the production of leukotrienes).

Liposomes (also known as lipid vesicles) are colloidal particles that are prepared from polar lipid molecules derived either from natural sources or chemical synthesis. Such spherical, closed structures composed of curved lipid bilayers, are typically used to entrap drugs, which are often cytotoxic, in order to reduce toxicity and/or increase efficacy. Liposome-entrapped drug preparations are often provided in a dry (e.g. freeze-dried) form, which is subsequently reconstituted with an aqueous solution immediately prior to administration. This is done in order to minimise the possibility of leakage of e.g. cytotoxic drug into aqueous solution and thereby reducing the entrapping effect of the liposome.

Liposomes have also been employed to encapsulate various drug compounds for delivery via the nasal route, in order to improve bioavailability or as an adjuvant. Drugs that may be mentioned include tetanus toxoid vaccine, insulin, desmopressin and diphenhydramine hydrochloride (see Türker et al, Review Article: Nasal Route and Drug Delivery Systems, Pharm. World ScL5 2004; 26, 137-142 and the references cited therein), as well as ciprofloxacin, CM3 and salbutamol (see Desai et al, A Facile Method of Delivery of Liposomes by Nebulization, J. Control. Release, 2002; 84, 69-78).

Liposome-entrapped cetirizine has been administered topically to evaluate peripheral antihistaminic activity and systemic absorption in a rabbit model (Elzainy et al, Cetirizine from Topical Phosphatidylcholine-Hydrogenated Liposomes, The AAPS Journal, 2004; 6, 1-7, see also Drug Development and Industrial Pharmacy, 2005; 31, 281-291).

Homogeneous pharmaceutical compositions containing cetirizine and a polar lipid liposome have been disclosed in international patent application WO 2005/107711.

The lipophilic behaviour of cetirizine in buffered aqueous phosphatidylcholine liposome systems has also been studied (Plemper van Balen G et al. , Lipophilicity behaviour of the zwitterionic antihistamine cetirizine in phosphatidylcholine liposomes/water systems, Pharm. Res. 2001; 18, 694-701).

Examples of other formulations comprising inter alia liposome-encapsulated active ingredients are discussed in US 4,427,649, EP 0249561, WO 00/38681, US 4,839,175 and WO 98/00111.
Iwanaga et al. (2000) Biological & Pharmaceutical Bulletin, Vol. 23 No. 3, 323 - 326 discloses a pharmaceutical composition comprising liposomes with egg phosphatidylcholine or synthetic phospholipids and the antihistamine diphenhydramine for nasal administration, and the use of said composition for the treatment of rhinitis.
Gursoy (1993) Pharmazie Vol. 48 No. 7, 549 - 550 describes a pharmaceutical composition comprising liposomes of egg yolk phosphatidylcholine or soy bean phosphatidylcholine, tocopherol and the antihistamine defibrotide for use in anti-inflammatory therapy.
WO 90/14105 discloses a pharmaceutical composition comprising liposomes of phosphatidylcholine, optionally dipalmitoylphosphatidylcholine, and the antihistamine diphenhydramine, or the anti-inflammatory substances codeine or pethidine.
US-A-5 569 464 discloses a pharmaceutical composition comprising liposomes of egg phosphatidylcholine and the antihistamine chlorpheniramine, and the anti-inflammatory agents dexamethasone, betamethasone or morphine.
Mishina et al. (1994) Pharmaceutical Research NY, US Vol. 11 No. 6, 848 - 854 discloses a pharmaceutical composition comprising liposomes of phosphatidylcholine and phosphatidylglycerol and the corticosteroid methylprednisolone.
US 2002/064524 discloses a pharmaceutical composition comprising liposomes of soy bean phosphatidylcholine and corticosteroids for the treatment of anti-inflammatory diseases.
US 4 427 649 discloses a pharmaceutical composition comprising liposomes of egg phosphatidylcholine and an anti-inflammatory steroid derivative bearing a lipophilic substituent for the treatment of inflammation.

Surprisingly, we have found that the irritation that may be associated with (e.g. nasal) administration of certain antiinflammatory and/or antihistaminic active ingredients may be reduced by way of use of a homogeneous pharmaceutical compositions comprising such active ingredients, a polar lipid liposome and a pharmaceutically acceptable carrier.

According to the invention, there is provided a homogeneous pharmaceutical composition for use in the treatment of an inflammatory disorder comprising an antiinflammatory and/or antihistaminic active ingredient, selected from the group comprising azelastine or a pharmaceutically-acceptable salt thereof, a steroid selected from alclometasone, beclometasone, budesonide, ciclesonide, clobetasol, clobetasone, deflazacort, dexamethasone, diflucortolone valerate, fluocinonide, fluocortolone, fluprednidene, flurometholone, fluticasone, halcinonide, hydrocortisone, mometasone, prednisolone, rimexolone, triamcinolone and a pharmaceutically- acceptable salt of any of these compounds in a pharmaceutically-acceptable aqueous carrier, and a polar lipid liposome, provided that the active ingredient is not cetirizine, wherein the concentration of active ingredient in the aqueous carrier is substantially similar, whether located inside or outside the liposomal structures and varies by +-20% when comparing concentrations inside and outside of the liposomal structures at room temperature and atmospheric pressure, which compositions are referred to hereinafter as "the compositions of the invention".

The skilled person will appreciate that antiinflammatory and/or antihistaminic active ingredients are employed in compositions of the invention in a pharmacologically-effective amount (*vide infra*). The term "pharmacologically-effective amount" refers to an amount of the antiinflammatory and/or antihistaminic active ingredient, which is capable of conferring the desired therapeutic effect on a treated patient, whether administered alone or in combination with another active ingredient. Such an effect may be objective (i.e. measurable by some test or marker) or subjective (i.e. the subject gives an indication of, or feels, an effect).

By "pharmaceutical compositions" we include compositions that are suitable for use in direct administration to mammals, and especially humans. In this respect, the term is intended to encompass formulations that include only components that are regarded in the art as suitable for administration to mammalian, and especially human, patients. In the context of the present invention, the term may also mean that the compositions of the invention are in a form of a liquid that is ready-to-use, directly from the shelf, and not a formulation in which drug is encapsulated inside liposomes requiring reconstitution shortly prior to administration in order to avoid leakage of drug from liposomes into an aqueous carrier.

By "homogeneous" we include not only that the compositions of the invention comprise liposomes dispersed evenly throughout the aqueous carrier, but further that the active ingredient is distributed throughout the whole composition. This means that, following formation of a mixture comprising liposomes and drug in aqueous medium, drug that is not encapsulated within liposome is not removed following liposome formation. This may, in the case of certain compositions of the invention, result in a substantially similar concentration of active ingredient in the relevant aqueous medium, whether that medium is located inside or outside of the liposomal structures. By "substantially similar", we include that the concentration varies by +-20% and particularly about +-10% (when comparing concentrations inside and outside of the liposomal structures) at room temperature and atmospheric pressure. Drug concentration profiles may be measured by standard techniques known to the skilled person, such as ³¹P-NMR. For example, a standard *in situ* probing technique, or a technique that involves separation of the liposomal fraction from the free aqueous carrier and measurement of the amount/concentration of drug associated with each fraction may be employed. Separation may be accomplished by centrifugation, dialysis, ultrafiltration, or gel filtration.

It is preferred that the compositions of the invention further include a pharmaceutically-acceptable buffer capable of providing a pH of from about pH 4 (e.g. 4.0) to about pH 8 (e.g. 8.0), preferably from about pH 5 (e.g. 5.0) to about pH 7 (e.g. 7.0). Appropriate buffers include those that will not interfere with the formation of liposomes, such as a phosphate (e.g. disodium phosphate, dipotassium phosphate, sodium dihydrogen phosphate, potassium dihydrogen phosphate or phosphoric acid plus base), citrate (e.g. sodium citrate or citric acid plus base), or acetate buffer (e.g. sodium acetate or acetic acid plus base), which is capable of maintaining a pH within the above-specified ranges. Buffers may be employed in an amount that is suitable to provide for the above-mentioned effects and such will be appreciated by the skilled person without recourse to inventive input. Appropriate quantities are for example in the range of about 1 mg/mL to about 30 mg/mL.

The term "inflammatory disorder" will be understood by those skilled in the art to include any condition characterised by a localised or a systemic protective response, which may be elicited by physical trauma, infection, chronic diseases, such as those mentioned hereinbefore, and/or chemical and/or physiological reactions to external stimuli (e.g. as part of an allergic response). Any such response, which may serve to destroy, dilute or sequester both the injurious agent and the injured tissue, may be manifest by, for example, heat, swelling, pain, redness, dilation of blood vessels and/or increased blood flow, invasion of the affected area by white blood cells, loss of function and/or any other symptoms known to be associated with inflammatory conditions.

The term will thus also be understood to include any inflammatory disease, disorder or condition per se, any condition that has an inflammatory component associated with it, and/or any condition characterised by inflammation as a symptom, including inter alia acute, chronic, ulcerative, specific, allergic and necrotic inflammation, and other forms of inflammation known to those skilled in the art. The term thus also includes, for the purposes of this invention, inflammatory pain, pain generally and/or fever.

Accordingly, compositions of the invention may be useful in the treatment of asthma, chronic obstructive pulmonary disease, pulmonary fibrosis, inflammatory bowel disease, irritable bowel syndrome, inflammatory pain, fever, migraine, headache, low back pain, fibromyalgia, myofascial disorders, viral infections {e.g. influenza, common cold, herpes zoster, hepatitis C and AIDS), bacterial infections, fungal infections, dysmenorrhea, burns, surgical or dental procedures, malignancies (e.g. breast cancer, colon cancer, and prostate cancer), hyperprostaglandin E syndrome, classic Bartter syndrome, atherosclerosis, gout, arthritis, osteoarthritis, juvenile arthritis, rheumatoid arthritis, fever, ankylosing spondylitis, Hodgkin's disease, systemic lupus erythematosus, vasculitis, pancreatitis, nephritis, bursitis, conjunctivitis, iritis, scleritis, uveitis, wound healing, dermatitis, eczema, psoriasis, stroke, diabetes mellitus, neurodegenerative disorders such as Alzheimer's disease and multiple sclerosis, autoimmune diseases, allergic disorders, rhinitis, ulcers, coronary heart disease, sarcoidosis and any other disease with an inflammatory component.

Compositions of the invention find particular utility in the treatment of rhinitis, migraine, acute pain, chronic pain and asthma. The term "rhinitis" will be understood to include any irritation and/or inflammation of the nose, whether allergic or non-allergic, including seasonal rhinitis (e.g. caused by outdoor agents such as pollen; hay fever) and/or perennial rhinitis (e.g. caused by house dust mites, indoor mold etc.), as well as the symptoms thereof.

The term "antiinflammatory and/or antihistaminic active ingredient" will be understood by the skilled person to include azelastine, alclometasone, beclometasone, budesonide, ciclesonide, clobetasol, clobetasone, deflazacort, dexamethasone, diflucortolone valerate, fluocinonide, fluocortolone, fluprednidene, flurometholone, fluticasone, halcinonide, hydrocortisone, mometasone, prednisolone, rimexolone, triamcinolone and a pharmaceutically-acceptable salt of any of these compounds.

Active ingredients may be employed in combination.

Any pharmaceutically-acceptable salt of an antiinflammatory and/or antihistammic active ingredient, as well as the free base form thereof may be used in the manufacture of compositions of the invention. Preferred salts include acetate salts, acetonate salts, aluminium salts, ammonium salts, arginine salts, bromide salts, butyrate salts, calcium salts, chloride salts, choline salts, citrate salts, diethanolamine salts, diethylamine salts, dipropionate salts, embonate salts, ethanolamine salts, ethylenediamine salts, formate salts, fumarate salts, fuorate salts, hydrobromide salts, hydrochloride salts, imidazole salts, lactate salts, lysine salts, magnesium salts, malate salts, maleate salts, malonate salts, meglumine salts, mesilate salts, morpholine salts, nitrate salts, phosphate salts, piperazine salts, potassium salts, propionate salts, sodium salts, succinate salts, sulfate salts, tartrate salts, teoclate salts, para-toluenesulfate salts, triethanolamine salts, triethylamine salts, valerate salts, etc. and/or as described in "Handbook of Pharmaceutical Salts", Eds. Stahl and Wermuth, Wiley, 2002, Chapter 12.

The amount of an antiinflammatory and/or antihistaminic active ingredient, or salt thereof that may be employed in preparation of compositions of the invention may be determined by the physician, or the skilled person, in relation to what will be most suitable for an individual patient. This is likely to vary with the nature of the active ingredient employed, the severity of the condition that is to be treated, as well as the species, age, weight, sex, renal function, hepatic function and response of the particular patient to be treated. It is preferred however that the compositions of the invention comprise an antiinflammatory and/or antihistaminic drug, or a salt thereof in an amount of from about 0.1 mg/mL to about 200 mg/mL calculated on the free-base form.

The total amount of active ingredient that may be present may be sufficient to provide a daily dose of drug per unit dosage that is appropriate for the active ingredient(s) that is/are employed. For example, this may be in the range about 20 µg to about 200 mg. The skilled person will appreciate that compositions of the invention may be dosed once or more times daily in one or more administrations in order to provide the aforementioned daily dose. Preferred ranges include from about 0.1 mg/mL to about 100 (e.g. about 70) mg/mL and, more particularly from about 0.2 mg/mL to about 50 mg/mL.

The above-mentioned dosages are exemplary of the average case; there can, of course, be individual instances where higher or lower dosage ranges are merited, and such are within the scope of this invention.

The term "liposome" will be well understood by those skilled in the art to include a structure consisting of one or more concentric spheres of polar lipid bilayers separated by water or aqueous buffer compartments.

Liposomes may be prepared by various methods using solvents, reduced pressure, two-phase systems, freeze drying, sonication etc. described, for instance, in Liposome Drug Delivery Systems, Betageri G V et al., Technomic Publishing AG, Basel, Switzerland, 1993.

The term "polar lipid" will be well understood by the skilled person to include any lipid with a polar head-group and two fatty acid residues, which is capable of forming liposomes.

Polar lipids, such as those described hereinafter, may be of a natural and/or a synthetic/semi-synthetic origin. Mixtures of natural and synthetic/semi-synthetic polar lipids may also be employed in compositions of the invention.

Polar lipids that may be employed in compositions of the invention may thus be based on, for example, phospholipids, and in particular phosphatidylcholine (PC), phosphatidylglycerol (PG), phosphatidylinositol (PI), phosphatide acid (PA), phosphatidylserine (PS), or mixtures thereof.

Phospholipids that may be employed in compositions of the invention comprise polar and non-polar groups linked to a backbone entity carrying hydroxyl groups, such as glycerol.

Phospholipids may also be represented by the general formula I 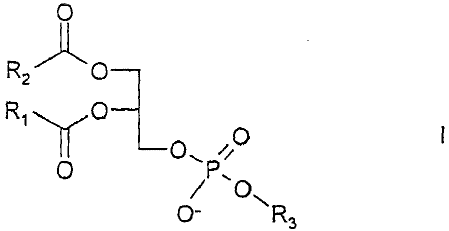 wherein R1 and R2 independently represent a saturated or unsaturated (e.g. alkenyl), branched or straight chain alkyl group having between 7 and 23 carbon atoms, preferably between 11 and 19 carbon atoms; and R3 represents an amide or ester bonding group, such as
-CH₂-CH(OH)-CH₂OH (phosphatidylglycerol),
-CH₂-CH₂-N(CH₃)₃ (phosphatidylcholine),
-CH₂-CH₂-NH₂ (phosphatidylethanolamine),
-H (phosphatidic acid), or
-CH₂CH(NH₂)-COOH (phosphatidylserine).

The phospholipid may be of natural origin. Natural phospholipids are preferably membrane lipids derived from various sources of both vegetable (e.g. rapeseed, sunflower, etc., or, preferably, soybean) and animal origin (e.g. egg yolk, bovine milk, etc.). Phospholipids from soybean, a major source of vegetable phospholipids, are normally obtained from the by-products (i.e. lecithins) in the refining of crude soybean oil by the degumming process. The lecithins are further processed and purified using other physical unit operations, such as fractionation and/or chromatography. Other phospholipids may be obtained, for example, by pressing various suitable seeds and grains, followed by solvent extraction and then further processing as described above. Phospholipids of natural origin that may be mentioned include for example those that are available under the tradenames Lipoid S75, Lipoid S100 and Lipoid S75-3N (Lipoid GmbH, Germany), which are all blends of several different phospholipids that are found in soybean.

The phospholipid may alternatively be of synthetic or semi-synthetic origin (i.e. prepared by chemical synthesis). For example, a multi-step chemical synthetic approach may be used in order to obtain the key phospholipid intermediates, 1,2-diacylglycerol, from (S)-1,2-isopropylideneglycerol, the latter providing the glycerol backbone that is characteristic of phospholipids. 1,2-Diacetylated phospholipids may then be obtained when the corresponding polar head group is attached via chemical synthesis to the 1,2-diacylglycerol intermediate. Generally, however, the origin of glycerol and the fatty acids used in the various steps may be of both natural and synthetic origin. Synthetic and/or semi-synthetic phospholipids that may be mentioned include dilaurylphosphatidylcholine (DLPC), dimyristolphosphatidylcholine (DMPC), dipalmitoylphosphatidylcholine (DPPC), dilaurylphosphatidylglycerol (DLPG), dimyristolphosphatidylglycerol (DMPG), dioleoylphosphatidylcholine (DOPC) and dioleoylphosphatidylglycerol (DOPG).

The polar lipid may alternatively comprise or, more preferably, consist of a glycolipid. In the context of the present invention, the term "glycolipid" designates a compound containing one or more monosaccharide residues bound by a glycosidic linkage to a hydrophobic moiety such as an acylglycerol, a sphingoid or a ceramide (N-acylsphingoid).

A glycolipid may be a glycoglycerolipid. In the context of the present invention, the term "glycoglycerolipid" designates a glycolipid containing one or more glycerol residues. According to a preferred aspect of the invention, the glycoglycerolipid comprises, or consists of, galactoglycerolipid, more preferably a digalactosyldiacylglycerol of the general formula II, 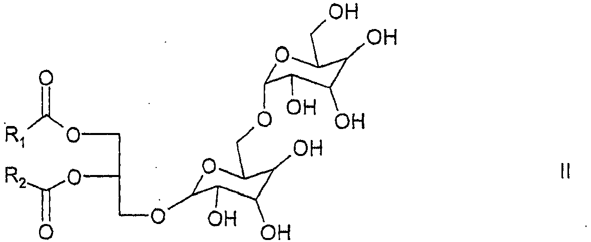 wherein R1 and R2 are as hereinbefore defined.

The glycolipid may alternatively be a glycosphingolipid. In the context of the present invention, the term "glycosphingolipid" designates a lipid containing at least one monosaccharide residue and either a sphingoid or a ceramide. The term may thus comprise neutral glycophingolipids, such as mono- and oligoglycosylsphingoids as well as oligo- and, more preferably, monoglycosylceramides. The term additionally comprises acidic glycosphingolipids such as sialoglycosphingolipids, uronoglycosphingolipids, sulfoglycosphingolipids, phosphoglycosphingolipids, and phosphonoglycosphingolipids. The glycosphingolipid can be ceramide, monohexosylcerarnide, dihexosylceramide, sphingomyelin, lysosphingomyelin, sphingosine, or a mixture thereof. Preferably the glycosphingolipid is sphingomyelin or products derived therefrom. The sphingomyelin content is preferably established by chromatographic methods. Sphingomyelin may be extracted from milk, preferably bovine milk, brain, egg yolk or erythrocytes from animal blood, preferably sheep. For the avoidance of doubt, synthetic and semi-synthetic sphingolipids are comprised by the invention.

The glycolipid may alternatively be a glycophosphatidylinositol. In the context of the present invention, the term "glycophosphatidylinositol" designates a glycolipid containing saccharides glycosidically linked to the inositol moiety of phosphatidylinositols.

Preferred glycolipids include digalactosyldiacylglycerol (DGDG).

It is preferred that the polar lipid is based on a phospholipid and, more particularly, a phospholipid derived from soybean (e.g. Lipoid S100, Lipoid S75 or Lipoid S75-3N).

Preferred polar lipids (such as phospholipids) are those that swell to a measurable degree in water and/or those which are capable of spontaneous liposome formation.

If the polar (e.g. phospho-) lipid does not swell spontaneously in water, the skilled person will appreciate that it is nevertheless possible to obtain liposomes by adding a more polar, swellable (e.g. phospho-) lipid, such as an anionic (e.g. phospho-) lipid (e.g. phosphatidylglycerol).

Liposome formation may be performed at above about 0°C (e.g. room temperature) if the phase transition temperature of the acyl chains (chain melting; gel-to-liquid crystals) is below the freezing point of water.

Whichever polar lipid substance (or combination thereof) is used, suitable total amounts/concentrations of lipid(s) that may be employed in preparation of a composition of the invention are in the range of about 10 mg/mL to about 120 mg/mL. Compositions of the invention that may be mentioned include those in which, when the polar lipid comprises phospholipid (whether in combination with another lipid or otherwise), the amount of phospholipid(s) in the composition is from about 10 (e.g. about 17, such as about 20) mg/mL to about 120 mg/mL. more preferably from about 25 (e.g. about 35) mg/mL to about 100 (e.g. about 70, such about 50, e.g. about 40) mg/mL. Typical ranges that may be mentioned include from about 25 (e.g. 27) mg/mL to about 50 mg/mL (e.g. 45 or, more particularly, 35 mg/mL). Further, the total amount of phospholipid (when the polar lipid comprises phospholipid) is preferably in the range from about 10 mg to about 80 mg (such as from about 17 (e.g. 20) mg to about 70 (e.g. 40) mg.

Compositions of the invention may also comprise an antioxidant, such as α-tocopherol, ascorbic acid, butylated hydroxyanisole, butylated hydroxytoluene, citric acid, fumaric acid, malic acid, monothioglycerol, propionic acid, propyl gallate, sodium ascorbate, sodium bisulfite, sodium metabisulfite, potassium metabisulfite, sodium sulfite, tartaric acid or vitamin E. Preferred antioxidants include butylated hydroxytoluene, α-tocopherol, ascorbic acid and butylated hydroxyanisole .

According to the invention a chelating agent may be used to reduce the metal ion catalysed oxidation of phospholipid and/or active ingredient(s). Examples of useful chelating agents are ethylenediaminetetraacetic acid (EDTA) and salts thereof (e.g. sodium or potassium EDTA), ethylenediaminetriacetic acid and diethylenetriaminepentaacetic acid (DTPA). It is also possible to use other agents that protect the composition of the invention and, in particular, any unsaturated fatty acid residues that may be present therein, from oxidation. Preferred chelating agents include EDTA and salts thereof.

The composition of the invention can comprise one or more preservatives. Examples of common preservatives for liquid pharmaceutical compositions are benzalkonium chloride, benzoic acid, butylated hydroxyanisole, butylparaben, chlorbutanol, ethylparaben, methylparaben, propylparaben, phenoxyethanol or phenylethyl alcohol. Preferred preservatives include benzalkonium chloride. Other preservatives that may be mentioned include sorbic acid.

In order to retain the composition of the invention at its application site it may also comprise viscosity-increasing agent such as, for instance, hydrophilic polymers like polyethyleneglycol, or crosslinked polyvinylpyrrolidone and/or cellulose derivatives such as hydroxypropylmethyl cellulose. Viscosity-increasing agents may also function as protective colloids to physically stabilise the composition of the invention prior to administration. Preferred protective colloids include hydroxypropylmethyl cellulose and, more particularly, polyethylene glycol.

Compositions of the invention may also comprise flavourings (e.g. lemon, menthol or peppermint powder) and/or sweeteners (e.g. neohesperidin).

Compositions of the invention may also comprise tonicity-modifying agents, such as sodium chloride, potassium chloride, glycerol, glucose, dextrose, sucrose, mannitol.

Optional additives, including buffering agents, preservatives, viscosity-increasing agents, antioxidants, tonicity-modifying agents and chelating agents should be selected, in terms of their identity and the amounts employed, keeping in mind that their detrimental effect on liposome stability should be kept at a minimum. For a given agent this can be ascertained by simple experiments, which are well within the understanding of the skilled person. Suitable amounts of such ingredients are however in the range about 0.01 mg/mL to about 10 mg/mL. It is preferred that the compositions of the invention contain at least one preservative, antioxidant, chelating agent, buffering agent and/or viscosity-increasing agent. Suitable amounts of any/all of these optional additives include from about 0.02 to about 5 (e.g. about 3) mg/mL (e.g. from about 0.1 to about 2 mg/mL

There is also disclosed a process for preparing compositions disclosed, which process comprises:
(a) mixing together (i) a polar lipid or a mixture of polar lipids that is/are swellable in aqueous media, (ii) an aqueous phase, and (iii) an antiinflammatory and/or antihistaminic active ingredient; and
(b) homogenising the preparation.

Aqueous phases as employed in step (a) above include water, or water in which something else is dissolved (i.e. an aqueous solution). Aqueous solutions may comprise e.g. buffer (*vide infra*). Aqueous solutions may also comprise an antiinflammatory and/or antihistaminic active ingredient (i.e. component (iii) above), in which case the polar lipid, or mixture of polar lipids is/are added to an aqueous solution of an antiirrfiammatory and/or antihistaminic active ingredient in step (a) above.

Step (a) of the above-mentioned process is preferably carried out in the presence of suitable agitation (e.g. stirring).

Preferably the pH of the preparation is adjusted, for example prior to the homogenisation step (b) above, to a desired value within the range of from about pH 4 (e.g. 4.0) to about pH 8 (e.g. 8.0), preferably from about pH 5 (e.g. 5.0) to about pH 7 (e.g. 7.0), by adding an acid or a base (e.g. hydrochloric acid and/or sodum hydroxide at an appropriate concentration (e.g. 1M)).

Preferably water, saline or buffer solution is added, for example prior to the homogenisation step (b) above and/or after the pH adjusting step mentioned above, to the preparation to obtain a desired final batch volume.

Solutions/liquids may be purged with nitrogen or argon at a suitable stage in the above process, if and as appropriate.

In the context of the present invention, a lipid may be said to be swellable in aqueous media if, when placed in contact with such a medium, it swells to a measurable degree.

Buffers may preferably be added to the aqueous solution of drug (and/or drug may be added to an aqueous buffer solution) prior to the addition of lipid.

The formation of the liposomes of the invention may be facilitated by the spontaneous swelling of the polar lipid in water forming a lamellar liquid crystalline phase having a maximum water content of about 35% by weight or higher depending on the nature of the polar lipid. Depending on the lipid or lipid mixture used and other conditions, spontaneous formation of liposomes may be achieved when excess water is added to this lamellar phase. If spontaneous formation is not achieved, the formation of liposomes may be accomplished by the mechanical dispersion step (i.e. the homogenisation step (b) of the above process) of the lamellar liquid-crystalline phase in excess water.

Homogenisation/dispersion methods include vigorous mechanical mixing or high speed homogenisation, for instance by means of an Ultra Turrax® (Jankel & Kühnke, Germany). Shaking, vortexing and rolling may also be performed as part of the homogenisation step of the above process.

A homogeneous size distribution of the liposomes of the invention may be desirable and may be obtained by extrusion through a membrane filter, such as one made of polycarbonate, with a pore size of about 100 nm. Membrane filters may be procured from Avestin Inc., Canada.

A reduced average liposome size and narrowed liposome size distribution may preferably also be obtained when the liposomal dispersion is subjected to highpressure homogenisation with a suitable homogeniser (Rannie APV, type 7.30 VH, Rannie AS, Denmark) at, for example, between about 300 bar and about 1000 bar, such as between about 400 bar and about 900 bar, e.g. about 500 to about 800 bar for between about 4 and about 8 (e.g. 7, such as 6) cycles.

We have found that the presence of certain active ingredients may result in a reduction of liposome size. Smaller liposomes are generally advantageous because they are more stable physically and, due to their higher surface area/volume ratio, are more easily resorbed by the mucosa.

We prefer that the diameter of liposomes in compositions of the invention is less than about 200 nm (e.g. between about 40 to about 100 nm), as measured by, for example, laser diffraction or dynamic light scattering, e.g. as described hereinafter.

Furthermore, the above-mentioned process for the preparation of compositions does not normally require conventional treatment with organic solvents such as chloroform or dichloromethane. However, if two or more membrane lipids are used it may be appropriate and/or necessary to treat them with organic solvent prior to the addition of the aqueous solvent. For example, the lipids may be dissolved in a volatile solvent or solvent mixture, such as chloroform or chloroform/methanol. The solution may then be deposited on the surfaces of a round-bottomed flask as the solvent is removed by rotary evaporation under reduced pressure. Au excess volume of aqueous buffer containing the drug may then be added to the dry thin film of lipids, which may then be allowed to swell to form liposomes. In other cases, if the active ingredient is significantly insoluble in water and/or phospholipid, it may be necessary to dissolve it and the phospholipid in an organic solvent prior to addition of the aqueous phase. Again, organic solvent may be removed (e.g. in *vacuo*) prior to addition of the aqueous phase.

Although compositions of the invention may be administered by any known route, including parenterally, topically and/or perorally, they may normally be administered transmucosally and, more particularly, nasally, ocularly and pulmonarily. For example, compositions of the invention may be administered by way of a nasal spray, nasal drops and/or eye drops. It is also possible to administer compositions of the invention as a fine mist to the lungs by nebulization. For nasal administration, any state-of-the-art device suitable for producing sprays of aqueous liposomal dispersions may be used.

Such formulations may be prepared in accordance with standard and/or accepted pharmaceutical practice.

Wherever the word "about" is employed herein in the context of dimensions (e.g. pH values, sizes, temperatures, pressures, etc.) and amounts (e.g. amounts, weights and/or concentrations of individual constituents in a composition or a component of a composition, proportions of drug inside/outside the liposomal structures, absolute doses of active ingredient, etc.), it will be appreciated that such variables are approximate and as such may vary by +- 10%, for example +-5% and preferably +- 2% (e.g. +- 1%) from the numbers specified herein.

The compositions of the invention, and .the above-mentioned process that may be employed for their preparation, have the advantages that are mentioned hereinbefore. In particular, compositions of the invention may reduce the incidence of inconvenient side-effects (and in particular irritation) that are often observed with e.g. nasally-administered formulations.

Compositions of the invention are easy to manufacture and enable the production of liposomal-based formulations that are in a ready-to-use form, avoiding the need for reconstitution prior to administration.

Compositions of the invention may also have the advantage that they may be prepared using established pharmaceutical processing methods and employ materials that are approved for use in foods or pharmaceuticals or of like regulatory status.

The invention is illustrated by way of the following examples.

General procedure. For weights and volumes reference is made to the tables below. A buffer solution is prepared by dissolving anhydrous citric acid and solid sodium hydroxide in 160 mL water (80% of the total batch volume) in a 200 mL volumetric flask. The weighed amount of active agent is added and dissolved by stirring with a magnetic stirrer. The phospholipid is separately weighed and added to the solution. Stirring, is continued until a well dispersed suspension has formed, the pH of which is adjusted to pH 5.0 +- 0.1 with 1.0 M NaOH and/or 1.0 M HCl. The volume of the preparation is then brought to the final batch volume of 200 mL. The preparation is transferred to a high pressure homogeniser (Rannie APV5 type 7.30 VH, Rannie AS, Denmark) and homogenised at 500-800 bar for 5 cycles. Aliquots of the thus obtained composition are removed from the collecting vessel and transferred to glass vials.

The above procedure is/was employed in order to prepare final compositions as outlined by Examples 1 to 8 below. Where appropriate, the quantities of the components are/were scaled up appropriately (e.g. in the case of Examples 1 to 5 , multiplied by 200). The procedure for Example 6 is described separately below.

### Example 1

| | |
|---|---|
| Budesonide | 1.3 mg |
| Phospholipid (soybean; Lipoid S100; Lipoid GmbH, Germany) | 35.0 mg |
| Benzalkonium chloride | 0.1 mg |
| Butylated hydroxytoluene (BHT) | 0.1 mg |
| Hydroxypropylmethylcellulose (Metolose 60SH-50) | 10 mg |
| Citric acid | 19.2 mg |
| Sodium hydroxide | 8.4 mg |
| 1 M HCl and/or 1 M NaOH | to pH 5.5 |
| Water for injection | to 1 mL |

### Example 2

| | |
|---|---|
| Fluticasone propionate | 0.5 mg |
| Phospholipid (soybean; Lipoid S100; Lipoid GmbH, Germany) | 17.5 mg |
| Phospholipid (DMPC; Lipoid GmbH, Germany) | 17.5 mg |
| Benzalkonium chloride | 0.1 mg |
| Butylated hydroxytoluene (BHT) | 0.1 mg |
| Citric acid | 19.2 mg |
| Sodium hydroxide | 8.4 mg |
| 1 M HCl and/or 1 M NaOH | to pH 5.5 |
| Water for injection | to 1 mL |

### Example 3

| | |
|---|---|
| Azelastine | 0.9 mg |
| Phospholipid (soybean; Lipoid S100; Lipoid GmbH, Germany) | 23.3 mg |
| Phospholipid (DMPC; Lipoid GmbH, Germany | 11.7 mg |
| Benzalkonium chloride | 0.1 mg |
| Butylated hydroxytoluene (BHT) | 0.1 mg |
| Polyethylene glycol (Macrogol 6000) | 10 mg |
| Citric acid | 19.2 mg |
| Sodium hydroxide | 8.4 mg |
| 1 M HCl and/or 1 M NaOH | to pH 5.5 |
| Water for injection | to 1 mL |

### Example 4

| | |
|---|---|
| Budesonide | 1.3 mg |
| Phospholipid (soybean; Lipoid S100; Lipoid GmbH, Germany) | 35.0 mg |
| Benzalkonium chloride | 0.2 mg |
| Butylated hydroxytoluene (BHT) | 0.2 mg |
| Citric acid | 19.2 mg |
| Sodium hydroxide | 8.4 mg |
| 1 M HCl and/or 1 M NaOH | to pH 5.0 |
| Water for injection | to 1 mL |

### Example 5

| | |
|---|---|
| Fluticasone propionate | 0.5 mg |
| Phospholipid (soybean; Lipoid S100; Lipoid GmbH, Germany) | 27.0 mg |
| Phospholipid (DMPC; Lipoid GmbH, Germany) | 8.0 mg |
| Sorbic acid | 1.0 mg |
| Na EDTA | 0.1 mg |
| Butylated hydroxytoluene (BHT) | 0.2 mg |
| Citric acid | 19.2 mg |
| Sodium hydroxide | 8.4 mg |
| 1 M HCl and/or 1 M NaOH | pH 5.0 |
| Water for injection | to 1 mL |

### Example 6

The commercially available nasal antihistamine azelastine (registered under trade names such as Azelvin®, Azosin®, Astelin®, Lastin® and Rhinolast®) was formulated using the quantities and steps outlined below.
1. 160 mL azelastine solution for nasal administration (Lastin®) containing 0.9 mg/mL azelastine was transferred into a 200 mL volumetric flask.
2. 7 g Soybean phospholipid (Lipoid S100; Lipoid GmbH, Germany) was added and the mixture was allowed to swell overnight.
3. The volume was brought to 200 mL by the addition of more azelastine solution (see step 1 above).
4. The pH was checked.
5. The solution was homogenised for 7 cycles at 800 bar as described in the general procedure above.

## Claims

1. A homogeneous pharmaceutical composition for use in the treatment of an inflammatory disorder comprising an antiinflammatory and/or antihistaminic active ingredient selected from the group comprising azelastine or a pharmaceutically-acceptable salt thereof, a steroid selected from alclometasone, beclometasone, budesonide, ciclesonide, clobetasol, clobetasone, deflazacort, dexamethasone, diflucortolone valerate, fluocinonide, fluocortolone, fluprednidene, flurometholone, fluticasone, halcinonide, hydrocortisone, mometasone, prednisolone, rimexolone, triamcinolone and a pharmaceutically- acceptable salt of any of these compounds in a pharmaceutically-acceptable aqueous carrier, and a polar lipid liposome, provided that the active ingredient is not cetirizine, wherein the concentration of active ingredient in the aqueous carrier is substantially similar, whether located inside or outside the liposomal structures and varies by +-20% when comparing concentrations inside and outside of the liposomal structures at room temperature and atmospheric pressure.

2. A composition for use as claimed in Claim 1 which further includes a pharmaceutically-acceptable buffer capable of providing a pH of from pH 4 to pH 8.

3. A composition for use as claimed in Claim 2, wherein the pH range is pH 5 to pH 7.

4. A composition for use as claimed in Claim 2 or Claim 3, wherein the buffer is a phosphate, citrate or acetate buffer.

5. A composition for use as claimed in Claim 4, wherein the buffer is disodium phosphate, dipotassium phosphate, sodium dihydrogen phosphate, potassium dihydrogen phosphate, phosphoric acid plus base, sodium citrate, citric acid plus base, sodium acetate or acetic acid plus base.

6. A composition for use as claimed in any one of Claims 2 to 5, wherein the quantity of buffer is in the range of 1 mg/ml to 30 mg/ml.

7. A composition for use as claimed in any one of the preceding claims, wherein the polar lipid is of a natural origin, is of a synthetic/semi-synthetic origin, or comprises a mixture of the two.

8. A composition for use as claimed in any one of the preceding claims, wherein the polar lipid comprises or consists of a phospholipid or a mixture of phospholipids.

9. A composition for use as claimed in Claim 8, wherein the phospholipid comprises one that is based on phosphatidylcholine, phosphatidylglycerol, phosphatidylinositol, phosphatidic acid, phosphatidylserine or a mixture thereof.

10. A composition for use as claimed in Claim 8 or Claim 9, wherein the phospholipid comprises one that is represented by the general formula I, 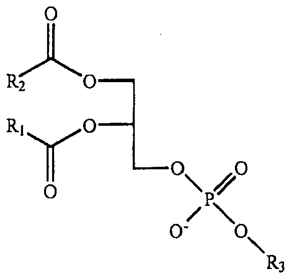
wherein R1 and R2 independently represent a saturated or unsaturated, branched or straight chain alkyl group having between 7 and 23 carbon atoms and R3 represents an amide or ester bonding group.

11. A composition for use as claimed in Claim 10 , wherein the amide or ester bonding group is -CH2-CH(OH)-CH2OH, -CH2-CH2-N(CHs)3, -CH2-CH2-NH2, -H or -CH2-CH(NH2)-COOH..

12. A composition for use as claimed in any one of Claims 8 to 11, wherein the phospholipid comprises a membrane lipid derived from soybean.

13. A composition for use as claimed in any one of Claims 8 to 12, wherein the phospholipid comprises dilaurylphosphatidylcholine, dimyristolphosphatidylcholine, dipalmitoylphosphatidylcholine, dilaurylphosphatidylglycerol, dimyristolphosphatidylglycerol, dioleoylphosphatidylcholine or dioleoylphosphatidylglycerol.

14. A composition for use as claimed in any one of Claims 1 to 7, wherein the polar lipid comprises or consists of a glycolipid or a mixture of glycolipids.

15. A composition for use as claimed in Claim 14, wherein the glycolipid comprises a glycoglycerolipid.

16. A composition for use as claimed in Claim 15, wherein the glycoglycerolipid comprises a galactoglycerolipid.

17. A composition for use as claimed in Claim 15, wherein the glycoglycerolipid comprises a digalactosyldiacylglycerol of the general formula II, 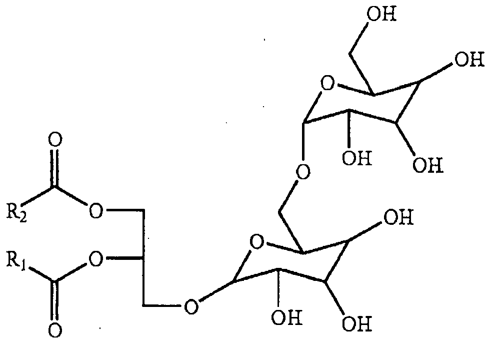
wherein R1 and R2 are as defined in Claim 10.

18. A composition for use as claimed in any one of Claims 14 to 17, wherein the glycolipid comprises digalactosyldiacylglycerol.

19. A composition for use as claimed in Claim 14, wherein the glycolipid comprises a glycosphingolipid.

20. A composition for use as claimed in Claim 19 wherein the glycosphingolipid comprises a monoglycosylsphingoid, an oligoglycosylsphingoid, an oligoglycosylceramide, a monoglycosylceramide, a sialoglycosphingolipid, a uronoglycosphingolipid, a sulfoglycosphingolipid, a phosphoglycosphingolipid, a phosphonoglycosphingolipid, a ceramide, a monohexosylceramide, a dihexosylceramide, a sphingomyelin, a lysosphingomyelin, a sphingosine or a mixture thereof.

21. A composition for use as claimed in Claim 20, wherein the glycosphingolipid comprises sphingomyelin or a product derived therefrom.

22. A composition for use as claimed in Claim 14, wherein the glycolipid comprises a glycophosphatidylinositol.

23. A composition for use as claimed in any one of the preceding claims, wherein the amount of polar lipid substance that is used is in the range of 10 mg/ml to 120 mg/ml.

24. A composition for use as claimed in any one of Claims 1 to 13 or 23, wherein the amount of phospholipid in the composition is from 17 mg/ml to 70 mg/ml.

25. A composition for use as claimed in Claim 24, wherein the amount is from 20 mg/ml to 40 mg/ml.

26. A composition for use as claimed in any one of the preceding claims, which further comprises an antioxidant, a chelating agent, a preservative or a viscosity-increasing agent.

27. A composition for use as claimed in Claim 26, wherein the antioxidant is alpha - tocopherol, ascorbic acid, butylated hydroxyanisole, butylated hydroxytoluene, citric acid, fumaric acid, malic acid, monothioglycerol, propionic acid, propyl gallate, sodium ascorbate, sodium bisulfite, sodium metabisulfite, potassium metabisulfite, sodium sulfite, tartaric acid and/or vitamin E; wherein the chelating agent is ethylenediaminetetraacetic acid (and/or a salt thereof), ethylenediaminetriacetic acid and/or diethylenetriaminepentaacetic acid; wherein the preservative is benzalkonium chloride, benzoic acid, butylated hydroxyanisole, butylparaben, chlorbutanol, ethylparaben, methylparaben, propylparaben, phenoxyethanol and/or phenylethyl alcohol; or wherein the viscosity-increasing agent is polyethyleneglycol, crosslinked polyvinylpyrrolidone and/or hydroxypropylmethyl cellulose.

28. A composition for use as claimed in any one of the preceding claims, wherein the diameter of the liposomes is less than 200 nm.

29. A composition for use as claimed in Claim 28, wherein the diameter is between 40 nm and 100 nm.

30. A composition for use as claimed in any one of Claims 1 to 29 wherein the composition is administered nasally.

31. A composition for use as claimed in any one of Claims 1 to 29, wherein the inflammatory disorder is rhinitis, asthma or inflammatory pain.

## Patentansprüche

1. Homogene pharmazeutische Zusammensetzung zur Verwendung in der Behandlung einer entzündlichen Erkrankung, umfassend einen entzündungshemmenden Wirkstoff und/oder Wirkstoff mit Antihistaminwirkung, ausgewählt aus der Gruppe umfassend Azelastin oder ein pharmazeutisch unbedenkliches Salz davon, ein Steroid, ausgewählt aus Alclometason, Beclometason, Budesonid, Ciclesonid, Clobetasol, Clobetason, Deflazacort, Dexamethason, Diflucortolonvalerat, Fluocinonid, Fluocortolon, Flupredniden, Flurometholon, Fluticason, Halcinonid, Hydrocortison, Mometason, Prednisolon, Rimexolon, Triamicinolon und ein pharmazeutisch unbedenkliches Salz von jeder dieser Verbindungen in einem pharmazeutisch unbedenklichen wässrigen Träger, sowie ein polares Lipidliposom, mit der Maßgabe, dass es sich bei dem Wirkstoff nicht um Cetirizin handelt, und wobei die Konzentration an Wirkstoff in dem wässrigen Träger im Wesentlichen ähnlich ist, egal, ob sie sich innerhalb oder außerhalb der Liposomenstrukturen befindet und beim Vergleich der Konzentrationen innerhalb und außerhalb der Liposomenstrukturen bei Raumtemperatur und Atmosphärendruck um +-20% schwankt.

2. Zusammensetzung zur Verwendung nach Anspruch 1, die weiterhin einen pharmazeutisch unbedenklichen Puffer beinhaltet, der fähig ist, einen pH-Wert von pH 4 bis pH 8 bereitzustellen.

3. Zusammensetzung zur Verwendung nach Anspruch 2, wobei der pH-Bereich pH 5 bis pH 7 ist.

4. Zusammensetzung zur Verwendung nach Anspruch 2 oder Anspruch 3, wobei es sich bei dem Puffer um einen Phosphat-, Citrat- oder Acetatpuffer handelt.

5. Zusammensetzung zur Verwendung nach Anspruch 4, wobei es sich bei dem Puffer um Dinatriumphosphat, Dikaliumphosphat, Natriumdihydrogenphosphat, Kaliumdihydrogenphosphat, Phosphorsäure plus Base, Natriumcitrat, Citronensäure plus Base, Natriumacetat oder Essigsäure plus Base handelt.

6. Zusammensetzung zur Verwendung nach einem der Ansprüche 2 bis 5, wobei die Puffermenge im Bereich von 1 mg/ml bis 30 mg/ml liegt.

7. Zusammensetzung zur Verwendung nach einem der vorhergehenden Ansprüche, wobei das polare Lipid natürlichen Ursprungs ist, synthetischen/halbsynthetischen Ursprungs ist oder eine Mischung der beiden umfasst.

8. Zusammensetzung zur Verwendung nach einem der vorhergehenden Ansprüche, wobei das polare Lipid ein Phospholipid oder eine Mischung von Phospholipiden umfasst oder daraus besteht.

9. Zusammensetzung zur Verwendung nach Anspruch 8, wobei das Phospholipid eines umfasst, das auf Phosphatidylcholin, Phosphatidylglycerin, Phosphatidylinosit, Phosphatidsäure, Phosphatidylserin oder einer Mischung davon beruht.

10. Zusammensetzung zur Verwendung nach Anspruch 8 oder Anspruch 9, wobei das Phospholipid eines umfasst, das der allgemeinen Formel I entspricht, 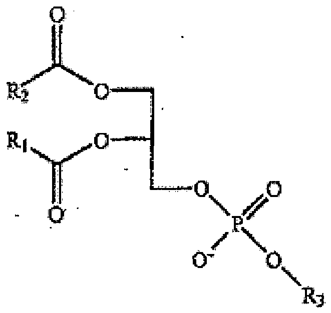
worin R₁ und R₂ unabhängig eine gesättigte oder ungesättigte, verzweigte oder geradkettige Alkylgruppe mit zwischen 7 und 23 Kohlenstoffatomen bedeutet und R₃ eine Amid- oder Esterbindungsgruppe bedeutet.

11. Zusammensetzung zur Verwendung nach Anspruch 10, wobei es sich bei der Amid- oder Esterbindungsgruppe um -CH2-CH(OH)-CH2OH, -CH2-CH2-N(CHs)3, -CH2-CH2-NH2, -H oder -CH2-CH(NH2)-COOH handelt.

12. Zusammensetzung zur Verwendung nach einem der Ansprüche 8 bis 11, wobei das Phospholipid ein aus Sojabohnen stammendes Membranlipid umfasst.

13. Zusammensetzung zur Verwendung nach einem der Ansprüche 8 bis 12, wobei das Phospholipid Dilaurylphosphatidylcholin, Dimyristolphosphatidylcholin, Dipalmitoylphosphatidylcholin, Dilaurylphosphatidylglycerin, Dimyristolphosphatidylglycerin, Dioleoylphosphatidylcholin oder Dioleoylphosphatidylglycerin umfasst.

14. Zusammensetzung zur Verwendung nach einem der Ansprüche 1 bis 7, wobei das polare Lipid ein Glycolipid oder eine Mischung von Glycolipiden umfasst oder daraus besteht.

15. Zusammensetzung zur Verwendung nach Anspruch 14, wobei das Glycolipid ein Glycoglycerolipid umfasst.

16. Zusammensetzung zur Verwendung nach Anspruch 15, wobei das Glycolgycerolipid ein Galactoglycerolipid umfasst.

17. Zusammensetzung zur Verwendung nach Anspruch 15, wobei das Glycoglycerolipid ein Digalactosyldiacylglycerin der allgemeinen Formel II umfasst, 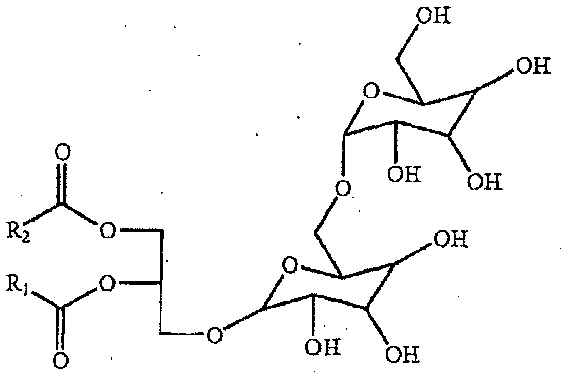
worin R₁ und R₂ wie in Anspruch 10 definiert sind.

18. Zusammensetzung zur Verwendung nach einem der Ansprüche 14 bis 17, wobei das Glycolipid Digalactosyldiacylglycerin umfasst.

19. Zusammensetzung zur Verwendung nach Anspruch 14, wobei das Glycolipid ein Glycosphingolipid umfasst.

20. Zusammensetzung zur Verwendung nach Anspruch 19, wobei das Glycosphingolipid ein Monoglycosylsphingoid, ein Oligoglycosylsphingoid, ein Oligoglycosylceramid, ein Monoglycosylceramid, ein Sialoglycosphingolipid, ein Uronoglycosphingolipid, ein Sulfoglycosphingolipid, ein Phosphoglycosphingolipid, ein Phosphonoglycosphingolipid, ein Ceramid, ein Monohexosylceramid, ein Dihexosylceramid, ein Sphingomyelin, ein Lysosphingomyelin, ein Sphingosin oder eine Mischung davon umfasst.

21. Zusammensetzung zur Verwendung nach Anspruch 20, wobei das Glycosphingolipid Sphingomyelin oder ein davon stammendes Produkt umfasst.

22. Zusammensetzung zur Verwendung nach Anspruch 14, wobei das Glycolipid ein Glycophosphatidylinosit umfasst.

23. Zusammensetzung zur Verwendung nach einem der vorhergehenden Ansprüche, wobei die Menge einer polaren Lipidsubstanz, die verwendet wird, im Bereich von 10 mg/ml bis 120 mg/ml liegt.

24. Zusammensetzung zur Verwendung nach einem der Ansprüche 1 bis 13 oder 23, wobei die Phospholipidmenge in der Zusammensetzung von 17 mg/ml bis 70 mg/ml beträgt.

25. Zusammensetzung zur Verwendung nach Anspruch 24, wobei die Menge von 20 mg/ml bis 40 mg/ml beträgt.

26. Zusammensetzung zur Verwendung nach einem der vorhergehenden Ansprüche, die weiterhin ein Antioxidationsmittel, einen Chelatbildner, ein Konservierungsmittel oder ein Verdickungsmittel umfasst.

27. Zusammensetzung zur Verwendung nach Anspruch 26, wobei es sich bei dem Antioxidationsmittel um alpha-Tocopherol, Ascorbinsäure, butyliertes Hydroxyanisol, butyliertes Hydroxytoluol, Citronensäure, Fumarsäure, Äpfelsäure, Monothioglycerin, Propionsäure, Propylgallat, Natriumascorbat, Natriumbisulfit, Natriummetabisulfit, Kaliummetabisulfit, Natriumsulfit, Weinsäure und/oder Vitamin E handelt; wobei es sich bei dem Chelatbildner um Ethylendiamintetraessigsäure (und/oder ein Salz davon), Ethylendiamintriessigsäure und/oder Diethylentriaminpentaessigsäure handelt; wobei es sich bei dem Konservierungsmittel um Benzalkoniumchlorid, Benzoesäure, butyliertes Hydroxyanisol, Butylparaben, Chlorbutanol, Ethylparaben, Methylparaben, Propylparaben, Phenoxyethanol und/oder Phenylethylalkohol handelt; oder wobei es sich bei dem Verdickungsmittel um Polyethylenglycol, vernetztes Polyvinylpyrrolidon und/oder Hydroxypropylmethylcellulose handelt.

28. Zusammensetzung zur Verwendung nach einem der vorhergehenden Ansprüche, wobei der Durchmesser der Liposomen weniger als 200 nm beträgt.

29. Zusammensetzung zur Verwendung nach Anspruch 28, wobei der Durchmesser zwischen 40 nm und 100 nm beträgt.

30. Zusammensetzung zur Verwendung nach einem der Ansprüche 1 bis 29, wobei die Zusammensetzung nasal verabreicht wird.

31. Zusammensetzung zur Verwendung nach einem der Ansprüche 1 bis 29, wobei es sich bei der entzündlichen Erkrankung um Rhinitis, Asthma oder Entzündungsschmerz handelt.

## Revendications

1. Composition pharmaceutique homogène pour utilisation dans le traitement d'un trouble inflammatoire comprenant une substance active anti-inflammatoire et/ou antihistaminique choisie dans le groupe comprenant l'azélastine ou un sel pharmaceutiquement acceptable de celle-ci, un stéroïde choisi parmi l'alclométasone, la béclométasone, le budésonide, le ciclésonide, le clobétasol, la clobétasone, le déflazacort, la dexaméthasone, le valérate de diflucortolone, le fluocinonide, la fluocortolone, le fluprednidène, la flurométholone, la fluticasone, l'halcinonide, l'hydrocortisone, la mométasone, la prednisolone, la rimexolone, la triamcinolone et un sel pharmaceutiquement acceptable de l'un quelconque de ces composés dans un véhicule aqueux pharmaceutiquement acceptable, et un liposome de lipide polaire, à condition que la substance active ne soit pas la cétirizine, la concentration de la substance active dans le véhicule aqueux étant sensiblement similaire, qu'elle soit située à l'intérieur ou à l'extérieur des structures liposomales et varie de +-20 % lorsqu'on compare les concentrations à l'intérieur et à l'extérieur des structures liposomales à température ambiante et à pression atmosphérique.

2. Composition pour utilisation selon la revendication 1 qui comprend en outre un tampon pharmaceutiquement acceptable capable de produire un pH de pH 4 à pH 8.

3. Composition pour utilisation selon la revendication 2, dans laquelle la plage de pH est de pH 5 à pH 7.

4. Composition pour utilisation selon la revendication 2 ou la revendication 3, dans laquelle le tampon est un tampon phosphate, citrate ou acétate.

5. Composition pour utilisation selon la revendication 4, dans laquelle le tampon est phosphate disodique, phosphate dipotassique, dihydrogénophosphate de sodium, dihydrogénophosphate de potassium, acide phosphorique plus base, citrate de sodium, acide citrique plus base, acétate de sodium ou acide acétique plus base.

6. Composition pour utilisation selon l'une quelconque des revendications 2 à 5, dans laquelle la quantité de tampon est dans la plage de 1 mg/ml à 30 mg/ml.

7. Composition pour utilisation selon l'une quelconque des revendications précédentes, dans laquelle le lipide polaire est d'une origine naturelle, est d'une origine synthétique/semi-synthétique, ou comprend un mélange des deux.

8. Composition pour utilisation selon l'une quelconque des revendications précédentes, dans laquelle le lipide polaire comprend ou est constitué d'un phospholipide ou un mélange de phospholipides.

9. Composition pour utilisation selon la revendication 8, dans laquelle le phospholipide comprend un phospholipide qui est à base de phosphatidylcholine, phosphatidylglycérol, phosphatidylinositol, acide phosphatidique, phosphatidylsérine ou un mélange de ceux-ci.

10. Composition pour utilisation selon la revendication 8 ou la revendication 9, dans laquelle le phospholipide comprend un phospholipide qui est représenté par la formule générale I, 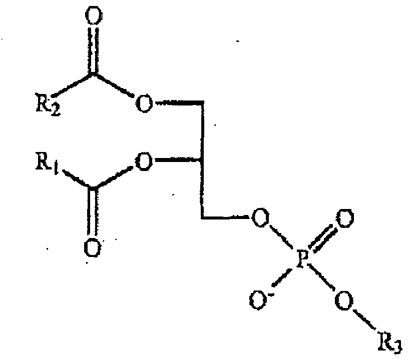
dans laquelle R₁ et R₂ représentent indépendamment un groupe alkyle saturé ou insaturé, ramifié ou à chaîne linéaire ayant entre 7 et 23 atomes de carbone et R₃ représente un groupe de liaison amide ou ester.

11. Composition pour utilisation selon la revendication 10, dans laquelle le groupe de liaison amide ou ester est -CH₂-CH(OH)-CH₂OH, -CH₂-CH₂-N(CH₂)₃, -CH₂-CH₂-NH₂, -H ou -CH₂-CH(NH₂)-COOH.

12. Composition pour utilisation selon l'une quelconque des revendications 8 à 11, dans laquelle le phospholipide comprend un lipide membranaire dérivé de soja.

13. Composition pour utilisation selon l'une quelconque des revendications 8 à 12, dans laquelle le phospholipide comprend la dilaurylphosphatidylcholine, la dimyristolphosphatidylcholine, la dipalmitoylphosphatidylcholine, le dilaurylphosphatidylglycérol, le dimyristolphosphatidylglycérol, la dioléoylphosphatidylcholine ou le dioléoylphosphatidylglycérol.

14. Composition pour utilisation selon l'une quelconque des revendications 1 à 7, dans laquelle le lipide polaire comprend ou est constitué d'un glycolipide ou un mélange de glycolipides.

15. Composition pour utilisation selon la revendication 14, dans laquelle le glycolipide comprend un glycoglycérolipide.

16. Composition pour utilisation selon la revendication 15, dans laquelle le glycoglycérolipide comprend un galactoglycérolipide.

17. Composition pour utilisation selon la revendication 15, dans laquelle le glycoglycérolipide comprend un digalactosyldiacylglycérol de formule générale II, 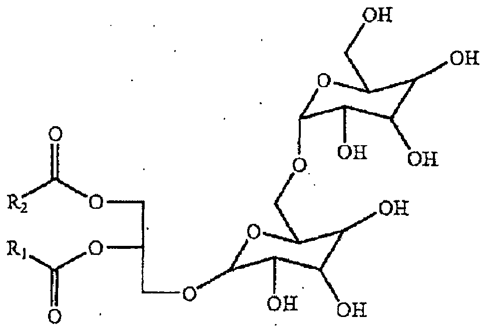
dans laquelle R₁ et R₂ sont tels que définis dans la revendication 10.

18. Composition pour utilisation selon l'une quelconque des revendications 14 to 17, dans laquelle le glycolipide comprend le digalactosyldiacylglycérol.

19. Composition pour utilisation selon la revendication 14, dans laquelle le glycolipide comprend un glycosphingolipide.

20. Composition pour utilisation selon la revendication 19 dans laquelle le glycosphingolipide comprend un monoglycosylsphingoïde, un oligoglycosylsphingoïde, un oligoglycosylcéramide, un monoglycosylcéramide, un sialoglycosphingolipide, un uronoglycosphingolipide, un sulfoglycosphingolipide, un phosphoglycosphingolipide, un phosphonoglycosphingolipide, un céramide, un monohexosylcéramide, un dihexosylcéramide, une sphingomyéline, une lysosphingomyéline, une sphingosine ou un mélange de ceux-ci.

21. Composition pour utilisation selon la revendication 20, dans laquelle le glycosphingolipide comprend la sphingomyéline ou un produit dérivé de celle-ci.

22. Composition pour utilisation selon la revendication 14, dans laquelle le glycolipide comprend un glycophosphatidylinositol.

23. Composition pour utilisation selon l'une quelconque des revendications précédentes, dans laquelle la quantité de substance de lipide polaire qui est utilisée est dans la plage de 10 mg/ml à 120 mg/ml.

24. Composition pour utilisation selon l'une quelconque des revendications 1 à 13 ou 23, dans laquelle la quantité de phospholipide dans la composition est de 17 mg/ml à 70 mg/ml.

25. Composition pour utilisation selon la revendication 24, dans laquelle la quantité est de 20 mg/ml à 40 mg/ml.

26. Composition pour utilisation selon l'une quelconque des revendications précédentes, qui comprend en outre un antioxydant, un agent chélateur, un conservateur ou un agent augmentant la viscosité.

27. Composition pour utilisation selon la revendication 26, dans laquelle l'antioxydant est l'alpha-tocophérol, l'acide ascorbique, l'hydroxyanisole butylé, l'hydroxytoluène butylé, l'acide citrique, l'acide fumarique, l'acide malique, le monothioglycérol, l'acide propionique, le gallate de propyle, l'ascorbate de sodium, le bisulfite de sodium, le métabisulfite de sodium, le métabisulfite de potassium, le sulfite de sodium, l'acide tartrique et/ou la vitamine E ; dans laquelle l'agent chélateur est l'acide éthylènediaminetétraacétique (et/ou un sel de celui-ci), l'acide éthylènediaminetriacétique et/ou l'acide diéthylènetriaminepentaacétique ; dans laquelle le conservateur est le chlorure de benzalkonium, l'acide benzoïque, l'hydroxyanisole butylé, le butylparabène, le chlorbutanol, l'éthylparabène, le méthylparabène, le propylparabène, le phénoxyéthanol et/ou l'alcool phényléthylique ; ou dans laquelle l'agent augmentant la viscosité est le polyéthylèneglycol, la polyvinylpyrrolidone réticulée et/ou l'hydroxypropylméthylcellulose.

28. Composition pour utilisation selon l'une quelconque des revendications précédentes, dans laquelle le diamètre des liposomes est inférieur à 200 nm.

29. Composition pour utilisation selon la revendication 28, dans laquelle le diamètre est compris entre 40 nm et 100 nm.

30. Composition pour utilisation selon l'une quelconque des revendications 1 à 29, la composition étant administrée par voie nasale.

31. Composition pour utilisation selon l'une quelconque des revendications 1 à 29, le trouble inflammatoire étant la rhinite, l'asthme ou la douleur inflammatoire.
